Europäisches Patentamt

European Patent Office

Office européen des brevets

(19)

(11) Veröffentlichungsnummer: **0 003 306**
**B1**

(12) # EUROPÄISCHE PATENTSCHRIFT

(45) Veröffentlichungstag der Patentschrift:
**30.12.81**

(21) Anmeldenummer: **79100108.4**

(22) Anmeldetag: **15.01.79**

(51) Int. Cl.³: **C 07 C 69/44,** C 07 C 67/38, C 07 C 69/34

(54) **Verfahren zur Herstellung von Butandicarbonsäureestern.**

(30) Priorität: **21.01.78 DE 2802580**

(43) Veröffentlichungstag der Anmeldung:
**08.08.79 Patentblatt 79/16**

(45) Bekanntmachung des Hinweises auf die Patenterteilung:
**30.12.81 Patentblatt 81/52**

(84) Benannte Vertragsstaaten:
**BE DE FR GB IT NL**

(56) Entgegenhaltungen:
**FR-A-2 384 738**

(73) Patentinhaber: **BASF Aktiengesellschaft,
Carl-Bosch-Strasse 38, D-6700 Ludwigshafen (DE)**

(72) Erfinder: **Kummer, Rudolf, Dr., Kreuzstrasse 6,
D-6710 Frankenthal 5 (DE)**
Erfinder: **Schneider, Heinz-Walter, Dr., Bruesseler
Ring 43, D-6700 Ludwigshafen (DE)**
Erfinder: **Weiss, Franz-Josef, Dr., Schlehdornweg 69,
D-6940 Weinheim (DE)**

## Verfahren zur Herstellung von Butandicarbonsäureestern

Gegenstand der Erfindung ist ein Verfahren zur Herstellung von Butandicarbonsäureestern durch Umsetzen von Butadien oder Butadien enthaltenden Kohlenwasserstoffgemischen mit Kohlenmonoxid und Alkanolen in Gegenwart von Kobaltcarbonylkatalysatoren und tertiären Stickstoffbasen.

Aus der DE-A Nr. 2037782 ist ein Verfahren zur Herstellung von Adipinsäure bekannt, bei dem man Butadien mit Kohlenmonoxid und Wasser bei erhöhtem Druck und bei erhöhter Temperatur in Gegenwart von Rhodiumverbindungen als Katalysatoren umsetzt. Die hierbei erzielten Ausbeuten an Dicarbonsäuren ermutigen nicht, das Verfahren technisch durchzuführen. Nach einem anderen in der DE-B Nr. 1518216 beschriebenen Verfahren wird Butadien in Gegenwart von Kobaltcarbonyl und Pyridin und Wasser bei 430 bar und 210°C zu Dicarbonsäuren umgesetzt. Die hierbei erzielte Ausbeute beträgt 50 bis 70% der Theorie, bezogen auf Butadien. Ferner ist aus «Bulletin of the Chemical Society of Japan», Band 46, 1973, S. 524 bis 530 bekannt, dass man Adipinsäuredimethylester erhält durch Umsetzen von Butadien mit Kohlenmonoxid und Methanol in Gegenwart von Kobaltcarbonyl und Pyridin und anschliessende Carbonylierung des so erhaltenen Pentensäuremethylesters mit dem selben Katalysator, wobei in der zweiten Stufe die Temperatur auf 200°C erhöht wird. Die Ausbeuten an Adipinsäuredimethylester liegen jedoch nur zwischen 47 und 51%.

Es war deshalb die technische Aufgabe gestellt, bei der Carbonylierung von Butadien die Ausbeute an Butandicarbonsäureestern zu erhöhen und insbesondere die Effektivität der Carbonylierung des als Zwischenproduktes erhaltenen Pentensäureesters zu verbessern.

Diese Aufgabe wird gelöst in einem Verfahren zur Herstellung von Butandicarbonsäureestern, bei dem man

a) Butadien oder Butadien enthaltende Kohlenwasserstoffgemische mit Kohlenmonoxid und $C_1$- bis $C_4$-Alkanolen in Gegenwart von tertiäre Stickstoffbasen und Kobaltcarbonylkatalysatoren bei Temperaturen von 80 bis 150°C unter erhöhtem Druck umsetzt

b) anschliessend den grössten Teil der tertiären Stickstoffbasen sowie überschüssige Kohlenwasserstoffe abtrennt und

c) den so erhaltenen Pentensäureester in Gegenwart des im Reaktionsgemisch verbleibenden Katalysators und der restlichen Menge an tertiären Stockstoffbasen mit Kohlenmonoxid und $C_1$- bis $C_4$-Alkanolen bei Temperaturen von 140 bis 200°C und unter erhöhtem Druck zu Butandicarbonsäureestern umsetzt,

dadurch gekennzeichnet, dass in der Stufe c) mindestens 50% des Kobaltkatalysators nicht als $\pi$-Allylkomplex mit Butadien vorliegen.

Das neue Verfahren hat den Vorteil, dass es gelingt, ohne den Katalysator abzutrennen, die Ausbeuten an Butandicarbonsäureestern zu steigern und insbesondere die Stufe der Carbonylierung von Pentensäureestern zu Butandicarbonsäureestern effektiver zu gestalten.

Man geht von reinem Butadien-1,3 oder Butadien enthaltenden Kohlenwasserstoffgemischen aus. Solche Kohlenwasserstoffgemische enthalten z.B. neben Butadien gesättigte Kohlenwasserstoffe mit 3 bis 5 Kohlenstoffatomen und einfach ungesättigte Olefine mit 3 bis 5 Kohlenstoffatomen. Der Butadiengehalt soll in der Regel mehr als 10 Gew.% betragen. In der Technik werden insbesondere $C_4$-Schnitte als Ausgangsgemisch verwendet. Als solches seien alle Gemische von überwiegend unverzweigten $C_4$-Kohlenwasserstoffen definiert, die mehr als 10 Gew.% Butadien-1,3 (Butadien) und mehr als 15 Gew.% Butene enthalten. Je nach Provenienz liegen die einzelnen Komponenten in solchen Gemischen normalerweise in folgenden Anteilen vor:

Butadien
    10 bis 70, durchschnittlich 40 bis 60 Gew.%
Isobuten
    15 bis 40, durchschnittlich 20 bis 35 Gew.%
But-1-en
    10 bis 40, durchschnittlich 10 bis 25 Gew.%
But-2-en
    5 bis 20, durchschnittlich 5 bis 15 Gew.%
Butane
    1 bis 10, durchschnittlich 1 bis 10 Gew.%
Butine
    0,1 bis 3, durchschnittlich 0,1 bis 3 Gew.%.

Solche $C_4$-Schnitte fallen beispielsweise an bei der Dehydrierung von Butan oder Buten als Nebenprodukte bei der Äthylengewinnung durch thermische Spaltung (Cracken) von Leichtbenzin (Naphtha) oder höheren Kohlenwasserstoffschnitten.

Als geeignete $C_1$- bis $C_4$-Alkanole seien genannt Methanol, Äthanol, Propanol, Butanol, Isobutanol. Besonders bevorzugt wird Methanol verwendet. Vorteilhaft wendet man einen Überschuss an Alkanolen an, insbesondere 1,5 bis 5 mol je mol Butadien.

Die Umsetzung führt man vorzugsweise bei Temperaturen von 120 bis 140°C und Drücken von 600 bis 1200 bar durch. Je mol Butadien verwendet man in der Regel 0,01 bis 0,1 gAtom Kobalt in Form von Kobaltcarbonylkomplexen.

Kohlenmonoxid wird vorteilhaft im Überschuss, z.B. dem 1,5- bis 10-fachen der stöchiometrisch erforderlichen Menge, angewandt.

Geeignete tertiäre Stickstoffbasen haben vorteilhaft einen $PK_a$-Wert von 3 bis 11 mit der Massgabe, dass die tertiären Stickstoffbasen vorzugsweise niedriger sieden als der jeweils herzustellende Pentensäureester. Vorzugsweise verwendet man N-heterocyclische Verbindungen wie Pyridin ($PK_a$ 5,3), Methylpyridine wie 3-Picolin ($PK_a$ 6,0), Isochinolin ($PK_a$ 5,4), ferner Trialkylamine wie Trimethylamin ($PK_a$ 11,0). Besondere technische Bedeutung hat Pyridin erlangt. Besonders bewährt hat es sich, wenn man je mol Kobaltcarbonylkatalysator 20 bis 50 mol Pyridin anwendet.

Die in der Stufe a) verwendeten Kobaltkatalysatoren werden entweder in situ aus Kobaltsalzen, z.B. fettsauren Kobaltsalzen, wie Formiaten, Acetaten, Propionaten oder Butyraten, erzeugt. Vorteilhaft bringt man den Katalysator bereits als Kobaltcarbonyl ein. Insbesondere hat es sich bewährt, wenn man Kobaltcarbonylkatalysator gelöst in Butadien oder $C_4$-Schnitt in das Reaktionsgemisch einbringt. Eine solche Lösung erhält man beispielsweise durch Umsetzen einer wässrigen Lösung von fettsauren Kobaltsalzen mit einem Gemisch aus Kohlenmonoxid und Wasserstoff in Gegenwart von Aktivkohle bei einer Temperatur von 100 bis 170°C und einem Druck von 100 bis 400 bar. Aus der wässrigen Lösung wird das entstandene Kobaltcarbonyl dann mit Butadien oder $C_4$-Schnitt extrahiert.

Das in der Stufe a) erhaltene Reaktionsgemisch enthält neben nicht umgesetztem Butadien gegebenenfalls andere Kohlenwasserstoffe, tertiäre Stickstoffbasen, Kobaltcarbonylkatalysator, nicht umgesetzte Alkanole, die als Wertprodukte erzeugten Pentensäureester sowie Nebenprodukte wie Valeriansäureester, Vinylcyclohexen, Butenyl- und Butylketone sowie Polymerisate des Butadiens.

Ein wesentliches Merkmal der Erfindung ist es, dass in der Stufe c) mindestens 50%, vorteilhaft 80%, des Kobaltkatalysators nicht als π-Allylkomplex mit Butadien vorliegen. Dies lässt sich insofern leicht überprüfen, als bei unvollständigem Umsatz in der Stufe a), also in Anwesenheit von Butadien, das IR-Spektrum des Katalysators im wesentlichen durch die Kobaltcarbonylbanden bei 2010 und 2080 cm$^{-1}$ gekennzeichnet ist, während die Bande bei 1930 cm$^{-1}$ wenig ausgeprägt ist. Bei zunehmender Verminderung des Butadiengehaltes verschwinden die Banden bei 2010 und 2080 cm$^{-1}$, während die Carbonylbande bei 1930 cm$^{-1}$ zur Hauptcarbonylbande wird. Dies deutet darauf hin, dass sich der Kobaltcarbonylkomplex, der Butadien gebunden enthält, nahezu vollständig in einen reinen Kobaltcarbonylkomplex, der nurmehr geringe Mengen an Butadien gebunden enthält, umgewandelt hat.

Eine geeignete Zusammensetzung des Katalysators, der für die Stufe c) geeignet ist, erzielt man beispielsweise dadurch, dass man in der Stufe a) das eingesetzte Butadien mindestens zu 99 Gew.% umsetzt. Anschliessend werden aus dem so erhaltenen Reaktionsgemisch nach dem Entspannen die darin enthaltenen tertiären Stickstoffbasen zum grössten Teil sowie gegebenenfalls überschüssige Kohlenwasserstoffe abgetrennt. Die Abtrennung erfolgt beispielsweise durch Destillation oder andere Trennverfahren wie Extraktion. Vorteilhaft werden tertiäre Stickstoffbasen und gegebenenfalls überschüssige Kohlenwasserstoffe durch Destillation unter vermindertem Druck abgetrennt. Vorteilhaft entfernt man die tertiären Stickstoffbasen bis auf einen Gehalt von 2 bis 10 mol je mol Kobaltcarbonylkatalysator.

Da es häufig unzweckmässig ist, Butadien mindestens zu 99% umzusetzen, insbesondere dann, wenn man Butadien enthaltende Kohlenwasserstoff verwendet, erhält man einen in der Stufe c) wirksamen Katalysator dadurch, dass man aus den in der Stufe a) erhaltenen Reaktionsgemischen nach dem Entspannen das freie Butadien z.B. durch Destillation, gegebenenfalls mit weiteren vorhandenen Kohlenwasserstoffen, abtrennt. Hierbei wird jedoch darauf geachtet, dass die gesamte Menge an tertiären Stickstoffbasen im Reaktionsgemisch verbleibt. Anschliessend wird das Reaktionsgemisch bei einer Temperatur von 100 bis 160°C und einem Druck von 50 bis 200 bar mit Kohlenmonoxid behandelt. Die Behandlungsdauer beträgt in der Regel 5 bis 60 min. Hierbei wandelt sich der Kobaltcarbonylkatalysator in die für die Stufe c) geeignete Form um. Anschliessend an diese Nachbehandlung wird dann der grösste Teil der im Reaktionsgemisch enthaltenen tertiären Stickstoffbasen wie oben aufgeführt, bei zu der dort genannten Menge abgetrennt. Es ist erwähnenswert, dass bei der Destillation die Temperatur im Destillationssumpf 75°C nicht überschreiten soll, um eine Zersetzung des Kobaltkatalysators zu vermeiden.

Der im so erhaltenen Reaktionsgemisch verbleibende Pentensäureester wird mit Kohlenmonoxid und $C_1$- bis $C_4$-Alkanolen, nachdem gegebenenfalls eine entsprechende Menge Alkanole erneut zugegeben wurde, bei Temperaturen von 140 bis 200°C und unter Drücken von 100 bis 400 bar in Gegenwart des im Reaktionsgemisch vorhandenen Kobaltkatalysators und der tertiären Stickstoffbasen umgesetzt. Es versteht sich, dass, wie vorgehend erwähnt, je mol Kobaltkatalysator 2 bis 10 mol tertiäre Stickstoffbasen im Reaktionsgemisch anwesend sind. Vorteilhaft hält man Temperaturen von 150 bis 180°C und Drücke von 100 bis 400 bar ein. Die mitverwendete Menge an Alkanolen beträgt z.B. 1,5 bis 4 mol je mo Pentensäureester. Es hat sich ferner als vorteilhaft erwiesen, dem Kohlenmonoxid einige Volumenprozent Wasserstoff, z.B. 0,2 bis 45 Vol.% zuzugeben, um die Reaktionsgeschwindigkeit zu erhöhen. Nach dem Entspannen werden aus dem erhaltenen Reaktionsgemisch überschüssige Alkanole und freie tertiäre Stickstoffbasen abdestilliert. Die chemisch an den Katalysator gebundenen tertiären Stickstoffbasen (1 bis 2 mol pro gAtom Kobalt) werden dabei nicht abdestilliert. Um eine Zersetzung des Kobaltkomplexes unter Ausscheidung von metallischem Kobalt zu vermeiden, die unerwünscht ist, hat es sich als zweckmässig erwiesen, in den Sumpf der Kolonne einen langsamen Strom von Kohlenmonoxid oder Kohlenmonoxid enthaltenden Gasen einzuleiten.

Das verbleibende Katalysator, Butandicarbonsäureester und Nebenprodukte enthaltende Reaktionsgemisch wird mit Oxidationsmitteln, z.B. molekularen Sauerstoff oder solche enthaltende Gase, wie Luft, im wässrig saurem Medium vorteilhaft bei einem pH-Wert von 3 bis 6 vorteilhaft bei Temperaturen von 80 bis 160°C behandelt. Nach der Behandlung trennt man z.B. durch Dekantieren in eine organische und eine wässrige Phase.

Aus der organischen Phase erhält man durch fraktionierte Destillation restliche tertiäre Stickstoffbasen, nicht umgesetzte Pentensäureester, die wieder der Carbonylierung zugeführt werden, sowie ein Gemisch aus Butandicarbonsäureestern (80 bis 85 Gew.% Adipinsäureester, 11 bis 15 Gew.% 2-Methylglutarsäureester und 3 bis 6 Gew.% 2-Äthylbernsteinsäureester). Das Estergemisch kann zur Herstellung von Diolen oder Polyestern verwendet werden. Der aus dem Estergemisch durch fraktionierte Destillation erhältliche Adipinsäureester eignet sich zur Herstellung von Adipinsäure. Die Kobaltsalze und gegebenenfalls freie Säure enthaltende wässrige Phase wird vorteilhaft wieder als Ausgangslösung zur Herstellung von Kobaltkatalysatoren zurückgeführt. Erfindungsgemäss erhaltene Adipinsäureester bzw. Adipinsäure eignen sich zur Herstellung von Polymeren.

Das Verfahren nach der Erfindung sei in folgenden Beispielen veranschaulicht.

*Beispiel 1:*

a) In ein Hochdruckgefäss von 1,9 l Inhalt führt man von unten stündlich 310 ml eines $C_4$-Schnittes, der 43 Gew.% Butadien (1,57 mol) sowie 3,7 g Kobalt in Form von Kobaltcarbonylverbindungen enthält, zu. Ausserdem führt man stündlich 124 g (1,57 mol) Pyridin, 100 g Methanol (3,14 mol) und 60 Nl Kohlenmonoxid zu. Die Carbonylierung verläuft bei einer Temperatur von 140°C und 600 bar. Das am Kopf des Hochdruckgefässes abgenommene Produkt wird entspannt, wobei gasförmig neben überschüssigem Kohlenmonoxid überschüssige $C_4$-Kohlenwasserstoffe abgetrennt werden. Diese enthalten praktisch kein Butadien. Der Umsatz ist also, bezogen auf Butadien, quantitativ.

b) Unter vermindertem Druck, um den Katalysator zu schonen, werden von dem Austrag stündlich etwa 52 g Methanol und 100 g Pyridin sowie Kohlenwasserstoffe abdestilliert. Im Destillationssumpf hält man eine maximale Temperatur von 65°C ein.

c) Der so erhaltene Destillationssumpf, der 3,7 g Kobalt als Carbonylkomplex, 165 g (1,44 mol) Pentensäureester sowie 22,8 g (0,288 mol) Pyridin enthält, wird zusammen mit 92 g (2,88 mol) Methanol und 55 Nl Kohlenmonoxid, das 2 Vol.% Wasserstoff enthält, einem weiteren Hochdruckgefäss von 1,7 l Volumen kontinuierlich von unten zugeführt. Die Carbonylierung wird bei einer Temperatur von 170°C unter einem Druck von 150 bar durchgeführt. Eine gaschromatographische Analyse des anfallenden Austrages zeigt, dass 95 Gew.% des eingesetzten Pentensäuremethylesters umgesetzt sind, wobei die Selektivität des Adipinsäuredimethylesters 76 Gew.% beträgt.

*Vergleichsbeispiel*

Man verfährt wie in Beispiel 1, führt die Reaktion in der Stufe 1 a) jedoch durch Senken der Temperatur auf 120°C durch, wobei entsprechend der gaschromatographischen Analyse, bezogen auf Butadien, nur ein Umsatz von 80% erzielt wird. Nach ansonst völlig analoger Arbeitsweise ergibt der Austrag der Stufe c) nach gaschromatographischer Analyse einen Umsatz von 56 Gew.% des eingesetzten Pentensäuremethylesters mit einer Selektivität von 77 Gew.% zu Adipinsäuredimethylester.

Die Erniedrigung des Umsatzes an Pentensäuremethylester zeigt deutlich, dass der Katalysator nicht in ausreichendem Masse in die für die Stufe c) aktive Form umgewandelt wurde.

*Beispiel 2:*

a) Man verfährt wie in Beispiel 1, führt die Reaktion jedoch bei 130°C und 600 bar durch, so dass nur 90% des Butadiens umgesetzt sind. Nach dem Entspannen werden die $C_4$-Kohlenwasserstoffe und das nicht umgesetzte Butadien abgetrennt. Danach wird der Reaktionsaustrag in Gegenwart des gesamten Pyridins und Methanols in einem weiteren Hochdruckgefäss von 0,4 l Inhalt bei 140°C und 100 bar CO Druck 60 min behandelt. Dabei wird der Katalysator, der vorher quantitativ als π-Allylkomplex gebunden vorlag, zu über 95% in Butadiensfreies Kobaltcarbonyl umgewandelt.

b) Nach dem Entspannen auf Normaldruck werden unter vermindertem Druck stündlich etwa 100 g Pyridin und 52 g Methanol abdestilliert (Sumpftemperatur 65°C).

c) Der so erhaltene Destillationssumpf, der den Kobaltcarbonylkatalysator, 165 g (1,44 mol) Pentensäureester sowie 22,8 g (0,288 mol) Pyridin enthält, wird zusammen mit 92 g (2,88 mol) Methanol und 55 Nl Kohlenmonoxid einem weiteren Hochdruckgefäss von 1,7 l Inhalt kontinuierlich von unten zugeführt. Die Carbonylierung wird bei 170°C und 150 bar durchgeführt. Eine gaschromatographische Analyse des anfallenden Austrages zeigt, dass 98,5% des Pentensäuremethylesters umgesetzt sind, wobei die Selektivität des Adipinsäuredimethylesters 77,5% beträgt.

## Patentansprüche

1. Verfahren zur Herstellung von Butandicarbonsäureestern, bei dem man:

a) Butadien oder Butadien enthaltende Kohlenwasserstoffgemische mit Kohlenmonoxid und $C_1$- bis $C_4$-Alkanolen in Gegenwart von tertiären Stickstoffbasen und Kobaltcarbonylkatalysatoren bei Temperaturen von 80 bis 150°C unter erhöhtem Druck umsetzt,

b) den grössten Teil der tertiären Stickstoffbasen sowie ggf. überschüssige Kohlenwasserstoffe abtrennt, und

c) den so erhaltenen Pentensäureester in Gegenwart des im Reaktionsgemisch verbleibenden Katalysators und der restlichen Menge an tertiären Stickstoffbasen mit Kohlenmonoxid und $C_1$- bis $C_4$-Alkanolen bei Temperaturen von 140 bis 200°C und unter erhöhtem Druck zu Butandicarbonsäureestern umsetzt,

dadurch gekennzeichnet, dass in der Stufe c) mindestens 50% des Kobaltkatalysators nicht als π-Allylkomplex mit Butadien vorliegen.

2. Verfahren nach Anspruch 1, dadurch gekennzeichnet, dass man in der Stufe a) je mol Kobaltcarbonylkatalysator 20 bis 50 mol tertiäre Stickstoffbasen anwendet.

3. Verfahren nach den Ansprüchen 1 und 2, dadurch gekennzeichnet, dass man in der Stufe c) je mol Kobaltkatalysator 2 bis 10 mol tertiäre Stickstoffbasen anwendet.

4. Verfahren nach den Ansprüchen 1 bis 3, dadurch gekennzeichnet, dass man in der Stufe a) einen Umsatz, bezogen auf Butadien, von mindestens 99 mol% einhält.

5. Verfahren nach den Ansprüchen 1 bis 3, dadurch gekennzeichnet, dass man in der Stufe a) als tertiäre Stickstoffbasen Pyridin verwendet und nach Abtrennen der Kohlenwasserstoffe das verbleibende Gemisch unter Einhaltung eines Verhältnisses von 20 bis 50 mol Pyridin je mol Kobaltkatalysator bei einer Temperatur von 100 bis 160°C und unter einem Druck von 50 bis 200 bar mit Kohlenmonoxid behandelt.

## Claims

1. A process for the preparation of butanedicarboxylic acid esters by:

a) reacting butadiene or hydrocarbon mixtures containing butadiene with carbon monoxide and a $C_1$- to $C_4$-alkanol in the presence of a tertiary nitrogen base and a cobalt carbonyl catalyst at from 80° to 150°C under superatmospheric pressure,

b) removing the greater part of the tertiary nitrogen base together with any excess hydrocarbon, and

c) reacting the resulting pentenoic acid ester, in the presence of the catalyst remaining in the reaction mixture, and in the presence of the remaining amount of tertiary nitrogen base, with carbon monoxide and a $C_1$- to $C_4$-alkanol at from 140° to 200°C under superatmospheric pressure, to give the butanedicarboxylic acid ester, characterized in that, in stage c), at least 50% of the cobalt catalyst is not in the form of a $\pi$-allyl complex with butadiene.

2. A process as claimed in claim 1, characterized in that from 20 to 50 mol of tertiary nitrogen base are used per mol of cobalt carbonyl catalyst in stage a).

3. A process as claimed in claims 1 and 2, characterized in that from 2 to 10 mol of tertiary nitrogen base are used per mol of cobalt catalyst in stage c).

4. A process as claimed in claims 1 to 3, characterized in that a conversion, based on butadiene, of at least 99 mol % is mained in stage a).

5. A process as claimed in claims 1 to 3, characterized in that, in stage b), pyridine is used as tertiary nitrogen base, and the mixture remaining after removal of the hydrocarbons is treated with carbon monoxide under a pressure of from 50 to 200 bar, at from 100° to 160°C, whilst maintaining a molar ratio of pyridine to cobalt catalyst of 20 to 50:1.

## Revendications

1. Procédé de préparation d'esters d'acide butanedicarboxylique, consistant à:

a) faire réagir du butadiène ou des mélanges d'hydrocarbures comprenant du butadiène avec de l'oxyde de carbone et des alcanols en $C_1$ à $C_4$ en présence de bases azotées tertiaires et de catalyseurs au cobalt/carbonyle sous pression accrue et à des températures de 80 à 150°C,

b) séparer la majeure partie des bases azotées tertiaires et les hydrocarbures éventuellement en excès, et

c) transformer l'ester d'acide penténique formé en présence du catalyseur resté dans le mélange réactionnel et de la fraction restante des bases azotées tertiaires par réaction avec l'oxyde de carbone et des alcanols en $C_1$ à $C_4$ en esters d'acide butane-dicarboxylique, caractérisé en ce que, dans le stade c), 50% au moins du catalyseur au cobalt ne se trouvent pas sous la forme d'un complexe $\pi$-allyle avec le butadiène.

2. Procédé suivant la revendication 1, caractérisé en ce que, dans le stade a), l'on emploie 20 à 50 mol de bases azotées tertiaires par mole de catalyseur au cobalt/carbonyle.

3. Procédé suivant l'une des revendications 1 ou 2, caractérisé en ce que, dans le stade c), l'on emploie 2 à 10 mol de bases azotées tertiaires par mole de catalyseur au cobalt/carbonyle.

4. Procédé suivant l'une des revendications 1 à 3, caractérisé en ce que l'on maintient, dans le stade a), un taux de conversion rapporté au butadiène d'au moins 99% molaires.

5. Procédé suivant l'une des revendications 1 à 3, caractérisé en ce que l'on emploie, dans le stade b), la pyridine comme base azotée tertiaire et que, après la séparation des hydrocarbures, on traite le mélange restant avec l'oxyde de carbone sous une pression de 50 à 200 bars et à une température de 100 à 160°C, en maintenant une proportion de 20 à 50 mol de pyridine par mole de catalyseur au cobalt.